# EUROPEAN PATENT SPECIFICATION

(11) **EP 1 698 702 B1**
(45) Date of publication and mention of the grant of the patent: **22.02.2012**
(21) Application number: 05004550.9
(22) Date of filing: 02.03.2005
(51) Int. Cl.: C12N 15/81

(54) **Recombinant protein expression system**
Expression System für rekombinante Proteine
Système d'expression pour protéines recombinantes

(43) Date of publication of application: 06.09.2006
(73) Proprietor: PharmedArtis GmbH, 52074 Aachen (DE)
(72) Inventor: Gellißen, Gerd, 42489 Wülfrath (DE)
(74) Representative: von Kreisler Selting Werner

(56) References cited:
- WO-A-01/38510
- WO-A-97/42307
- GELLISSEN G ET AL: "HIGH-LEVEL EXPRESSION OF FOREIGN GENES IN HANSENULA POLYMORPHA" BIOTECHNOLOGY ADVANCES, ELSEVIER PUBLISHING, BARKING, GB, vol. 10, no. 2, 1992, pages 179-189, XP000914545 ISSN: 0734-9750
- GELLISSEN G: "Heterologous protein production in methylotrophic yeasts" APPLIED MICROBIOLOGY AND BIOTECHNOLOGY, SPRINGER VERLAG, BERLIN, DE, vol. 54, no. 6, December 2000 (2000-12), pages 741-750, XP002333889 ISSN: 0175-7598
- HOLLENBERG C P ET AL: "PRODUCTION OF RECOMBINANT PROTEINS BY METHYLOTROPHIC YEASTS" CURRENT OPINION IN BIOTECHNOLOGY, LONDON, GB, vol. 8, 1997, pages 554-560, XP000939179 ISSN: 0958-1669
- GELLISSEN G ET AL: "METHYLOTROPHIC YEAST HANSENULA POLYMORPHA AS PRODUCTION ORGANISM FOR RECOMBINANT PHARMACEUTICALS METHYLOTROPHE HEFE HANSENULA POLYMORPHA ALS PRODUKTIONSORGANISMUS FUER REKOMBINANTE THERAPEUTIKA" ARZNEIMITTEL FORSCHUNG. DRUG RESEARCH, ECV EDITIO CANTOR VERLAG, AULENDORF, DE, vol. 46, no. 9, 1996, pages 943-948, XP001156104 ISSN: 0004-4172
- GELLISEN G ET AL: "HETEROLOGOUS GENE EXPRESSION IN HANSENULA POLYMORPHA: EFFICIENT SECRETION OF GLUCOAMYLASE" BIO/TECHNOLOGY, NATURE PUBLISHING CO. NEW YORK, US, vol. 9, March 1991 (1991-03), pages 291-295, XP002062735 ISSN: 0733-222X
- GELLISSEN G ET AL: "GENE EXPRESSION IN METHYLOTROPHIC YEASTS" BIOPROCESSING TECHNOLOGY, ENGLEWOOD, US, vol. 22, 1995, pages 195-239, XP001013697 ISSN: 0885-5625

## Description

The present invention relates to a polynucleotide comprising functionally linked a nucleic acid sequence comprising an autonomously replicating sequence element; a nucleic acid sequence comprising a ribosomal DNA (rDNA) gene; a nucleic acid sequence encoding a selection marker; and a nucleic acid sequence comprising a nucleic acid sequence encoding a expressible polypeptide and a promoter nucleic acid sequence governing the expression of said expressible polypeptide. Moreover, the invention encompasses vectors, host cells or kits comprising said polynucleotide or methods for the manufacture of a protein or a pharmaceutical composition using said polynucleotide. Finally, the invention relates to collections of polynucleotides which can be assembled and a kit comprising said collections of polynucleotides.

Several yeast species have been developed as expression platforms for the production of recombinant proteins, among others *Saccharomyces cerevisiae, Hansenula polymorpha, Pichia pastoris, Arxula adeninivorans, Kluyveromyces lactis* and *Yarrowia lipolytica.* For all these systems individual sets of vectors and host cells exist. Though some of them are distinghuished by an industrial track record, all systems have limitations. Some limitations originate from deficiencies of the vectors. Vectors of an episomal fate, e.g., may result in unstable transformants, other targeting sequences may lead to inefficient gene expression, the vector size may lead to low transformation rates. The limitations are described in detail in Gellissen (Ed), Hansenula polymorpha - biology and applications, Wiley-VCH, Weinheim, 2002 or Gellissen (Ed), Production of recombinant proteins, Wiley-VCH, Weinheim 2004.

WO-A2-01/38510 discloses integration vectors for the targeted and stable integration of heterologous DNA into the genome of a host organism, especially a fungus, and to the use thereof. The invention also relates to a method for producing one or more recombinant proteins using the disclosed vectors. The aim of WO-A2-01/38510 is to provide integration vectors which enable foreign DNA to be integrated into the genome of a host organism in a stable manner and with a high copy number and if necessary, to enable different genes to be expressed for a simultaneous coproduction of different proteins. Another aim of WO-A2-01/38510 is provide stable production strains and a method for producing heterologous proteins. To this end, an integration vector comprising the following is provided: a) at least one rDNA sequence from yeast; b) at least one non-deficient selection marker gene; and c) at least one expression cassette, said expression cassette comprising a promoter element that is functional in the host organism, an area of a heterologous or genuine gene that code for a desired protein and a terminator element that is functional in the host organism. WO-A2-01/38510 also discloses a host organism containing at least one inventive vector. A preferred organism is the methylotrophic yeast *Hansenula polymorpha.*

G. Gellissen et al. in Biotech. Adv. Vol. 10., No. 2, (1992), pp. 179-189 discloses high-level expression of foreign genes in *Hansenula polymorpha.* It reports that the methylotrophic yeast *Hansenula polymorpha,* which belongs to a limited number of non-saccharomyces yeast species, has been used as hosts for heterologous gene expression. It has successfully been applied for the production of hormones, antigens and enzymes. The system excels by mitotically stable recombinant strains, high productivity and faithful processing of the produced polypeptides. The favourable characteristics of this microorganism for protein production at an industrial scale are described.

Thus, the technical problem underlying the present invention can be seen as the provision of an expression system useful for the efficient and reliable manufacture of recombinant polypeptides in yeast.
The technical problem is solved by the embodiments characterized in the claims and herein below.

Accordingly, the present invention relates to a polynucleotide comprising functionally linked:
(a) an autonomously replicating nucleic acid sequence element comprising a CEN element (GI number: 406851), a 2µm (micron) element (GI number: 3296) and a nucleic acid sequence being at least 70 % identical thereto;
(b) a nucleic acid sequence comprising a ribosomal DNA (rDNA) gene;
(c) a nucleic acid sequence encoding a selection marker; and
(d) a nucleic acid sequence comprising an expressible nucleic acid sequence encoding a polypeptide and a promoter nucleic acid sequence governing the expression of said expressible sequence.

The term "polynucleotide" encompasses DNA or RNA which may be in a circular or open conformation. The polynucleotides of the invention may be incorporated into vectors, artificial chromosomes or other DNA molecules. Moreover, the polynucleotides may be integrated into the genome of a host cell in copy numbers ranging from one copy to multiple copies.
The term "functionally linked" means that the nucleic acid sequences are linked in the polynucleotides of the invention as to allow functional interaction. A promoter, for instance, shall be positioned in the polynucleotide as to allow control of the expression of the expressible polynucleotide. The marker gene must be incorporated into the polynucleotide as to allow expression of the selection marker. The autonomously replicating sequence and the rDNA sequences must be incorporated in the polynucleotide in a manner which allows them to be biologically active, e.g. to allow autonomous replication of the polynucleotide. It is well known in the art how the said nucleic acid sequences can be incorporated in a polynucleotide to allow that all sequences can exert their biological function. The nucleic acid sequences are, preferably, linked together by ligation of the individual nucleic acid sequences having at their 5' and 3' ends suitable linker arms. The linker arms, which will appear as spacers between the nucleic acid sequences in the polynucleotide, may contain recognition sites for restriction enzymes. Preferably, said restriction enzymes are selected from the group consisting of *BcuI, Eco*47III, *Sal*I*, EcoR*I, or *Sac*II or any other rare cutting enzyme.
The term "autonomously replicating sequence" encompasses all nucleic acid sequences which are capable to initiate replication of a polynucleotide comprising such an autonomously replicating nucleic acid sequence. Autonomously replicating nucleic acid sequences are well known in the art. Preferred autonomously replicating sequences are described herein below. The term "ribosomal DNA gene" refers to nucleic acid sequences encoding genes for ribosomal RNA. Preferred rDNA sequences are described herein below.
The term "nucleic acid encoding a selection marker" encompasses nucleic acids which encode a polypeptide which, if produced by a cell, allows survival or growth of the cell. Preferably, a selection is carried out by exposing the cell to environmental conditions under which the cell will survive or grow only if the selection marker is present in the cell or produced by the cell. Such conditions can be easily created by including an agent which is detrimental for its survival or growth to the culture medium for a host cell. Suitable nucleic acid sequences for selection markers are described herein below.
The term "expressible nucleic acid sequence" refers to all nucleic acid sequences which encode polypeptides and which can be expressed in a cell including those encoding small peptides. The nucleic acid sequence encoding said expressible polypeptide may comprise merely coding sequence or may comprise additional non-coding sequences. The expression cassette (nucleic acid sequence for the expressible polypeptide and the promoter) of the polynucleotide of the invention also contains a ribosome binding site for translation initiation and a transcription terminator.
The term "promoter nucleic acid sequence" refers to a nucleic acid sequence which is responsible for the control of transcription of RNA from the nucleic acid encoding the expressible polypeptide. Such promoter sequences will comprise binding sites for the various transcription factors required for initiation of transcription and for enhancement of transcription. These transcription factor binding sites may include those for the so called basal transcription factors as well as those for specific enhancers. The promoter nucleic acid sequences referred to herein may be either promoter sequences which allow constitutive transcription or transcription which will appear after providing a suitable stimulation. Stimulation may be achieved by activation of a promoter or deactivation of a repression mechanism of a repressed promoter. A promoter may be kept in a repressed state, for instance, by including suitable agents into the culture medium of a cell. Stimulation of the promoter can be achieved by introducing the cell comprising the said promoter in a culture medium which lacks the repressing agent. Preferred promoter nucleic acid sequences are described herein below.
Surprisingly, it has been found in accordance with the present invention that a polynucleotide arranged as the polynucleotide of the present invention is particularly well suited for efficient polypeptide expression in cells, in particular, yeast cells. Thus, a strategy to overcome the problems stated in the prior art is to provide a vector which is a small a possible, a targeting sequence by which the heterologous DNA is targeted to a genome site of high expressibility and suitable selection markers. In the present invention a modular system of vectors with the said elements is provided. Advantageously, the modular construction of the polynucleotide of the invention allows that an expression cassette containing a gene of interest can be cloned using convenient, small and well established cloning vectors. Moreover, the present inventions provides means for the high yield expression of polypeptides in an industrial scale without having the drawbacks of the expression systems described in the prior art.

The definitions and explanations of the terms made hereinabove and below apply mutatis mutandis for all disclosed embodiments.

In light of the foregoing, in a preferred embodiment of the invention said autonomously replicating sequence is an ARS element.
The term "ARS element" refers to replication signal sequences. They can be obtained from various eukaryotic species, preferably, those of the sub family Saccharomycetoideae including *Hansenula polymorpha, Saccharomyces cerevisiae, Arxula adeninivorans, and Schwanniomyces occidentalis.*

More preferably, said ARS is selected from any one of HARS (GI number: 2777, X03540), SWARS (GI number: 9844090, AJ278886) or ARS1 (GI number: 4667, V01341) or has a nucleic acid sequence being at least 70 % identical thereto.
Preferably, the HARS sequence comprises nucleotides 6 to 500 flanked by a *Bcu*I linker following position 500 and a *Sac*II linker before position 6. Preferably, the SwARS sequence comprises nucleotides 8 to 1535 flanked by a *Bcu*I linker following position 1535 and a *Sac*II linker before position 8. Preferably, the ARS1 sequence comprises nucleotides 618 to 1362 flanked by a *Bcu*I linker following position 1362 and a SacII linker before position 618.
A nucleic acid sequence which is 70 % identical to another nucleic acid (reference sequence) contains 70 % of the nucleotide composition of the reference sequence. Such a sequence is a variant of a specific ARS mentioned before having the same biological function, i.e. acting as autonomously replicating element as described herein above. Preferably, the identical nucleotides in both sequences can be aligned to each other. Whether a nucleic acid sequence is, in accordance with the present invention, at least 70 % identical to another nucleic acid sequence can be determined by suitable algorithms available in the prior, including BLAST algorithms provided at the NCBI. More preferably, the variant nucleotide sequences referred to in accordance with the present invention are at least 80 %, at least 85 %, at least 90 %, at least 95 %, at least 97 %, at least 98 % or at least 99 % identical to one of the aforementioned ARS sequences. Such variant nucleic acid sequences, preferably, hybridize under stringent conditions to any one of the aforementioned specific ARS sequences. Stringent conditions in accordance with the present invention are hybridization in 2x SSC at 55 °C. This definition of variant sequences applies for all other embodiments referring to varaiant sequences, i.e. sequences being to a certain degree identical to a specific sequence, mutatis mutandis. However, it must be considered that the variant sequences of those other sequences will have other biological functions. The biological functions of the nucleic acid sequences referred to in the context of the present invention have been described already above.

According to the invention said autonomously replicating sequence is a CEN element (GI number: 406851), a 2µm (micron) element (GI number: 3296) or has a nucleic acid being at least 70 % identical thereto.
Preferably, the 2micron sequence comprises nucleotides 2406 to 3873 flanked by a *Bcu*I linker following position 3873 and a *Sac*II linker before position 1406. Preferably, the CEN sequence comprises nucleotides 3248-4911 flanked by a *Bcu*I linker following position 4911 and a *Sac*II linker before position 3248.

In accordance with the aforementioned embodiment, the invention relates in a further preferred embodiment to a polynucleotide wherein said rDNA gene is selected from any one of NTS2-ETS-18S-ITSI (SEQ ID NO: 1), 25S rDNA (SEQ ID NO: 2), 18S rDNA (SEQ ID NO: 3), NTS-18S (SEQ ID NO: 4), ITS-5S-ETS-18S-IST-5,8S-ITS (SEQ ID NO: 5) or has a nucleic acid sequence being at least 70 % identical thereto.
Preferably, SEQ ID 1 is flanked by a *Bcu*I linker following position 3056 and an *Eco*47III linker before position 1. Preferably, SEQ ID 2 is flanked by a *Bcu*I linker following position 7653 and an *Eco*47III linker before position 3772. Preferably, SEQ ID 3 is flanked by a *Bcu*I linker following position 3386 and an *Eco*47III linker before position 1622. Preferably, SEQ ID 4 is flanked by a *Bcu*I linker following position 3798 and an *Eco*47III linker before position 7. Preferably, SEQ ID 5 is flanked by a *Bcu*I linker following position 3542 and an *Eco*47III linker before position 520.

Yet in another preferred embodiment of the invention said selection marker is selected from the group consisting of *ALEU2* gene (GI number: 21436710), *AILV1 gene* (GI number: 2687748), *URA3 gene* (GI number: 406851), *hph-PHO5* gene, *kan* gene or *ATRP1* gene (SEQ ID NO: 6. Nucleotides 1-263 of SEQ ID NO: 6 correspond to the Promotor, nucleotides 264-980 represent the *ATRP1* open reading frame and nucleotides 981-1132 correspond to the Terminator sequence.).
Preferably, the *ALEU2* sequence comprises nucleotides 1 to 2187 flanked by an *Eco*47III linker following position 2187 and a *Sal*I linker before position 1. Preferably, SEQ ID 6 is flanked by an *Eco*47III linker following position 1132 and a *Sal*I linker before position 1. Preferably, the *AILV1* sequence comprises nucleotides 1 to 2746 flanked by an *Eco*47III linker following position 2746 and a *Sal*I linker before position 1. Preferably, the *URA3* sequence comprises nucleotides 1439 to 2600 flanked by an *Eco*47III linker following position 2600 and a *SalI* linker before position 1. *hph* and *kan* genes are also flanked by *Eco*47III and *Sal*I linkers.
Suitable techniques for selecting cells expressing these selection markers are described in the prior art and are well known to the person skilled in the art and are described, e.g., in Gellissen (Ed), Production of recombinant proteins, Wiley-VCH, Weinheim 2004. The selection mechanisms may comprise, for instance, auxotrophy complementation, antibiotic resistance or item and copy number amplification.

In a further preferred embodiment of the invention said promoter is selected from any one of *TEF1* (GI number: 620041), *FMD* (SQ ID NO: 7), *MOX* (SEQ ID NO: 8), *TPS* (SEQ ID NO: *9), AHSB4m* (GI number: 27550960), *GAA* (GI number: 600384), *ATAL* (GI number:17382031), *AACP50* (GI number: 17382027), or *ALIP* (GI number: 58651818 or has a nucleic acid sequence being at least 70 % identical thereto.
Preferably, the *TEF1* sequence comprises nucleotides 1 to 303 flanked by an *Eco*RI linker following position 303 and a *SalI* linker before position 1. Preferably, SEQ ID NO: 7 is flanked by an *Eco*RI linker following position 1086 and a *SalI* linker before position 1. Preferably, SEQ ID NO: 8 is flanked by an *Eco*RI linker following position 1502 and a *Sal*I linker before position 1. Preferably, SEQ ID NO: 9 is flanked by an *Eco*RI linker following position 564 and a SalI linker before position 1. Preferably, the *AHSB4* sequence comprises nucleotides 1 to 600 flanked by an *Eco*RI linker following position 600 and a *SalI* linker before position 1. Preferably, the *GAA* sequence comprises nucleotides 1 to 636 flanked by an *Eco*RI linker following position 636 and a *SalI* linker before position 1. Preferably, the *ATAL* sequence comprises nucleotides 1 to 515 flanked by an *Eco*RI linker following position 515 and a *SalI* linker before position 1. Preferably, the *AACP50* sequence comprises nucleotides 1 to 468 flanked by an EcoRI linker following position 468 and a *SalI* linker before position 1.

Preferably, the *ALIP* sequence comprises nucleotides 1 to 624 flanked by an *Eco*RI linker following position 624 and a *Sal*I linker before position 1.

The present invention preferably encompasses a polynucleotide, wherein said expressible nucleic acid sequence encodes a secreted protein.
A "secreted protein" in accordance with the present invention comprises, preferably, a secretion signal sequence or leader sequence. The protein or polypeptide, both terms may be used interchangeable, will be exported by the cell, i.e. it will be secreted into the culture medium. A secreted protein in accordance with the present invention may be either a protein having a naturally occurring leader sequence or a protein which has been fused to a leader sequence as to allow its secretion.

More preferably, said secreted protein is an enzyme, growth factor, cytokine, survival factor, clotting factor, cytokine or growth factor receptor, structural protein of the extracellular matrix.

Also, the present invention encompasses, preferably, a polnucleotide, wherein the nucleic acid sequences (a) to (d) are arranged in 5' to 3' orientation starting with (a) at the 5'end and ending with (d) at the 3'end of the said polynucleotide.
The resulting polynucleotide, thus, comprises from 5'to 3' a consecutive order of the nucleic acid sequences (a), (b), (c), and (d). The nucleic acid sequences may be separated by spacer sequences, e.g. for restriction sites.

The present invention also relates to a vector comprising the polynucleotide of the present invention.
The term "vector" encompasses cloning vectors or an expression vectors. The vector may be, for example, in the form of a plasmid, a cosmid, etc. Such vectors include chromosomal, nonchromosomal and synthetic DNA sequences, e.g., derivatives of SV40; bacterial plasmids; phage DNA; baculovirus; yeast plasmids; vectors derived from combinations of plasmids and phage DNA, viral DNA such as vaccinia, adenovirus, fowl pox virus, pseudorabies, and parts thereof. However, any other vector may be used as long as it is replicable and viable in the host cell. The appropriate nucleic acid sequence may be inserted into the vector by a variety of procedures. In general, the nucleic acid sequence is inserted into an appropriate restriction endonuclease site(s) by techniques known in the art. Such procedures and others are deemed to be within the scope of the person skilled in the art. The nucleic acid sequence encoding an expressible polypeptide in the expression vector is, as discussed above, operatively linked to an appropriate expression control sequence (promoter) to direct mRNA synthesis. In addition, the expression vectors preferably contain one or more selectable marker to provide a phenotypic trait for selection of transformed host. Large numbers of suitable vectors are known in the prior art, and are commercially available. The following vectors are provided by way of example. Bacterial: pQE70, pQE60, pQE-9 (Qiagen), pBS, pD10, phagescript, psiX174, pbluescript SK, pBSKS, pNH8A, pNH16a, pNH18A, pNH46A (Stratagene); ptrc99a, pKK223-3, pKK233-3, pDR540, pRIT5 (Pharmacia). Eukaryotic: pWLNEO, pSV2CAT, pOG44, pXT1, pSG (Stratagene) pSVK3, pBPV, PMSG, pSVL (Pharmacia). Other vectors are described in detail in Gellissen (Ed), Hansenula polymorpha - biology and applications, Wiley-VCH, Weinheim, 2002 or Gellissen (Ed), Production of recombinant proteins, Wiley-VCH, Weinheim 2004. These vectors may be used as entire plasmids or parts thereof may be used as vector backbones. However, any other plasmid or vector may be used as long as they are replicable and viable in the host cells.

Preferably, said vector has a nucleic acid sequence as shown in SEQ ID NO: 10.

The present invention further encompasses a host cell comprising the polynucleotide or the vector of the present invention.
The term "host cell" relates to cells which are capable of expressing the expressible polypeptide from the polynucleotide of the invention. Moreover, the cells are preferably capable of autonomously replicating the polynucleotide of the invention. The polynucleotide of the invention can be introduced into the host cells by standard techniques. Introduction of the construct into the host cell can be effected, e.g., by calcium phosphate transfection, DEAE-Dextran mediated transfection, or electroporation (Davis, L., Dibner, M., Battey, I., Basic Methods in Molecular Biology, (1986) or Gellissen (Ed), Hansenula polymorpha - biology and applications, Wiley-VCH, Weinheim, 2002.).

More preferably, the cell is a yeast cell, most preferably, *Hansenula polymorpha* or *Arxula adeninivorans.* This includes all strains and mutants of these species. Mutants may be nuturally occurring ones or those which have been generated by recombinant DNA techniques. Suitable techniques for obtaining such mutants are described, e.g., in Gellissen (Ed), Hansenula polymorpha - biology and applications, Wiley-VCH, Weinheim, 2002.

The present invention relates to a method for the manufacture of a polypeptide comprising the steps of:
(a) expressing the polynucleotide or the vector of the invention in the host cell of the invention; and
(b) recovering from the said host cell the polypeptide encoded by the expressible nucleic acid sequence.
The term "expressing" as used herein encompasses culturing of the cells under suitable conditions as to allow expression of the polypeptide of interest. The host cells are, preferably, cultured in a liquid medium which provides an environment suitable for high yield expression of the polypeptide. The culture conditions, such as temperature, pH and the like, are those previously used with the host cell selected for expression, and will be apparent to those of ordinarily skill in the art (see, e.g., Gellissen (Ed), Hansenula polymorpha - biology and applications, Wiley-VCH, Weinheim, 2002 or Gellissen (Ed), Production of recombinant proteins, Wiley-VCH, Weinheim 2004.).
The polypeptides may be recovered by techniques well known in the art. These techniques include purification by molecular sieve chromatography or absorption chromatography. To this ends, the proteins may be labeled by suitable absorption tags, such as 6His-tag, HA-tag, MYC-tag, FLAG-tag or similar short peptide motifs. The polypeptides may be purified by well known absorption techniques using biotin, avidin, and/or streptavidin. Antibody absorption is another alternative technique for purification.

Moreover, the present invention relates to a method for the manufacture of a pharmaceutical composition comprising the steps of the aforementioned method of the invention and the further step of formulating the produced polypeptide in a pharmaceutically acceptable manner.
The term "pharmaceutical composition" as used herein comprises the substances of the present invention and optionally one or more pharmaceutically acceptable carrier. The substances of the present invention may be formulated as pharmaceutically acceptable salts. Acceptable salts comprise acetate, methylester, HCI, sulfate, chloride and the like. The pharmaceutical compositions can be conveniently administered by any of the routes conventionally used for drug administration, for instance topically. The substances may be administered in conventional dosage forms prepared by combining the drugs with standard pharmaceutical carriers according to conventional procedures. These procedures may involve mixing, granulating and compressing or dissolving the ingredients as appropriate to the desired preparation. It will be appreciated that the form and character of the pharmaceutically acceptable carrier or diluent is dictated by the amount of active ingredient with which it is to be combined, the route of administration and other well-known variables. The carrier(s) must be "acceptable" in the sense of being compatible with the other ingredients of the formulation and not deleterious to the recipient thereof. The pharmaceutical carrier employed may be, for example, either a solid or liquid. Exemplary of solid carriers are lactose, terra alba, sucrose, talc, gelatin, agar, pectin, acacia, magnesium stearate, stearic acid and the like. Exemplary of liquid carriers are phosphate buffered saline solution, syrup, oil such as peanut oil and olive oil, water, emulsions, various types of wetting agents, sterile solutions and the like referred to above. Similarly, the carrier or diluent may include time delay material well known to the art, such as glyceryl mono-stearate or glyceryl distearate alone or with a wax. The pharmaceutical composition according to the present invention can be administered in various manners to achieve the desired effect. Said pharmaceutical compositions can be administered either alone or in the formulated as pharmaceutical preparations to the subject being treated either orally, topically or parenterally. Moreover, the substance can be administered in combination with other substances either in a common pharmaceutical composition or as separated pharmaceutical compositions. The diluent is selected so as not to affect the biological activity of the combination. Examples of such diluents are distilled water, physiological saline, Ringer's solutions, dextrose solution, and Hank's solution. In addition, the pharmaceutical composition or formulation may also include other carriers, adjuvants, or nontoxic, nontherapeutic, nonimmunogenic stabilizers and the like. A therapeutically effective dose refers to that amount of the substance according to the invention which ameliorate the symptoms or condition. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. The dosage regimen will be determined, as set forth before, by the attending physician based on clinical factors. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment.
A typical doses were specified above. The pharmaceutical compositions and formulations referred to herein are administered at least once in accordance with the use of the present invention. However, the said pharmaceutical compositions and formulations may be administered more than one time, for example from one to four times daily up to a non-limited number of days. Specific formulations according to the invention are prepared in a manner well known in the pharmaceutical art and usually comprise at least one active substance referred to herein above in admixture or otherwise associated with a pharmaceutically acceptable carrier or diluent thereof. For making those formulations the active substance(s) will usually be mixed with a carrier or diluted by a diluent, or enclosed or encapsulated in a capsule, sachet, cachet, paper or other suitable containers or vehicles. A carrier may be solid, semisolid, gel-based or liquid material which serves as a vehicle, excipient or medium for the active ingredients. Said suitable carriers comprise those mentioned above and others well known in the art, see, e.g., Remington's Pharmaceutical Sciences, Mack Publishing Company, Easton, Pennsylvania. The formulations can be adopted to the mode of administration comprising the forms of tablets, capsules, suppositories, solutions, suspensions or the like. The dosing recommendations will be indicated in product labeling by allowing the prescriber to anticipate dose adjustments depending on the considered patient group, with information that avoids prescribing the wrong drug to the wrong patients at the wrong dose.

The present invention furthermore encompasses a kit comprising the polynucleotide, the vector and/or the host cell of the present invention.
The kit of the present invention is preferably designed for carrying out the methods of the present invention. The kit may, accordingly, comprise a manual for carrying out the method of the invention. The components of the kit may be packaged together in separate vials.

Also, the present invention relates to a collection of vectors comprising a nucleic acid sequence comprising
(a) an autonomously replicating sequence element;
(b) a nucleic acid sequence comprising a ribosomal DNA (rDNA) gene;
(c) a nucleic acid sequence encoding a selection marker; and
(d) a nucleic acid sequence comprising an expressible nucleic acid sequence encoding a polypeptide and a promoter nucleic acid sequence governing the expression of said expressible sequence;
wherein said nucleic acid sequences can be assembled resulting in a polynucleotide comprising at least one, at least two or at least three of the nucleic acid sequences referred to in any one of (a) to (c) and the nucleic acid sequence referred to in (d).
The vectors of the collection include the aforementioned polynucleotides in a manner which allows modular construction of a polynucleotide of the present invention. The vectors are constructed as to allow excision of a nucleic acid sequence from one vector and cloning the said excised nucleic acid sequence in another vector of the collection using the same restriction sites and resulting in the order of the elements as described for the polynucleotide of the present invention.

Preferably, the nucleic acid sequences can be assembled by use of any one of the restriction enzymes *BcuI, Eco*47III, *SalI, EcoRI,* or *Sac*II.

Finally, the present invention relates to a kit comprising the collection of the invention and suitable means which allow assembling of the said nucleic acid sequences.

The figures show:

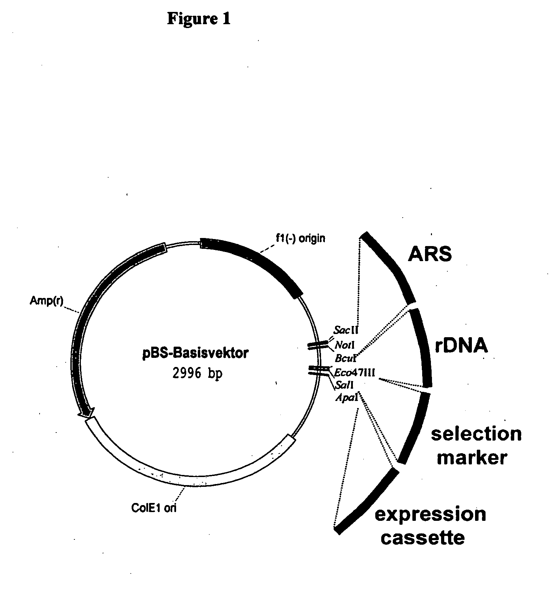

- **Figure 1:**: Schematic drawing of the polynucleotide of the invention (ARS, rDNA, selection marker, and expression cassette consisting of promoter and nucleic acid encoding the expressible polypeptide) comprised by the 2,996 bp pBS- vector. Restriction sites for endonucleases are indicated.

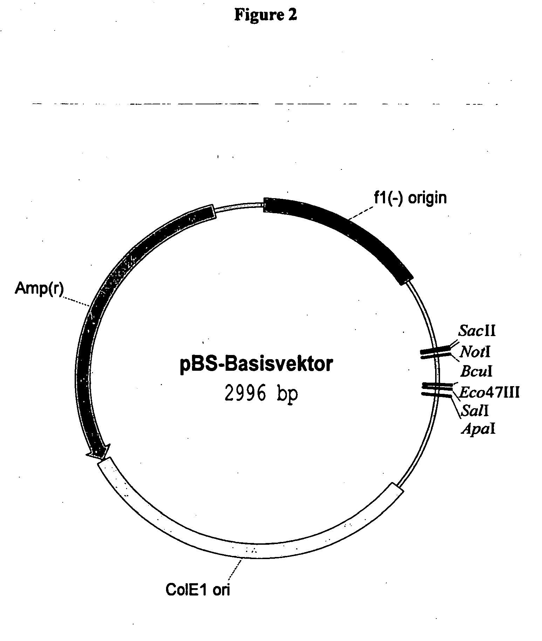

- **Figure 2:**: Schematic drawing of the vector without insertion of the polynucleotide of the invention.

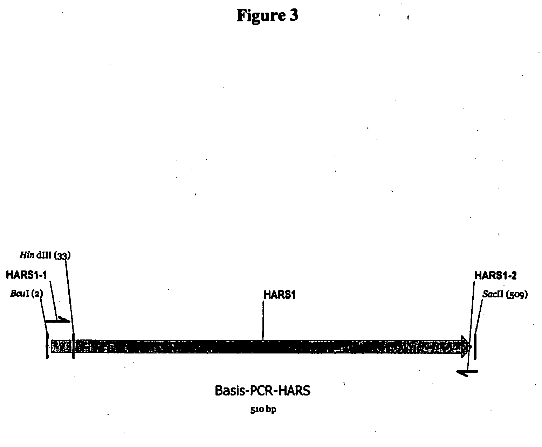

- **Figure 3:**: Schematic drawing of the HARS element. Restriction sites for endonucleases are indicated.

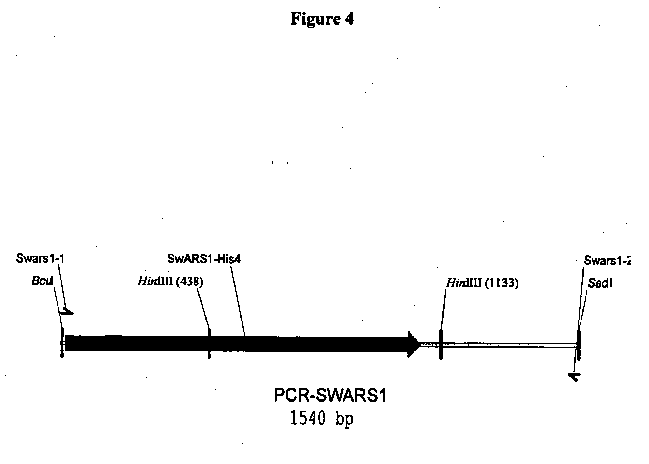

- **Figure 4:**: Schematic drawing of the SwARS element. Restriction sites for endonucleases are indicated.

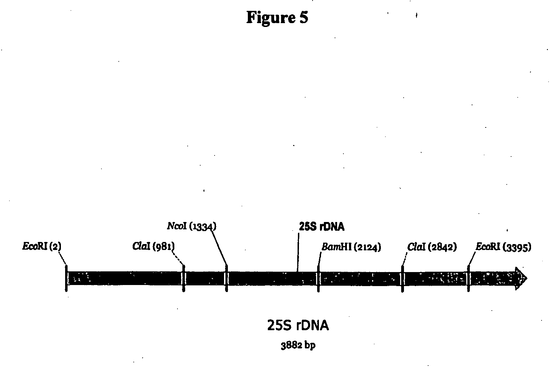

- **Figure 5:**: Schematic drawing of the 25S rDNA. Restriction sites for endonucleases are indicated.

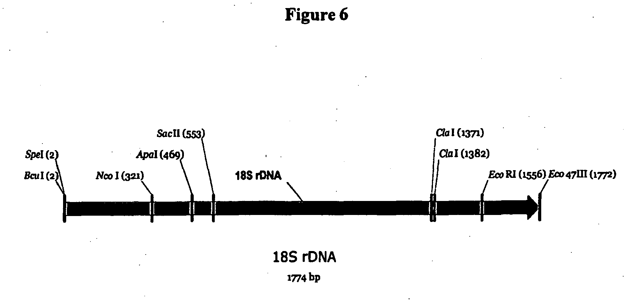

- **Figure 6:**: Schematic drawing of the 18SrDNA. Restriction sites for endonucleases are indicated.

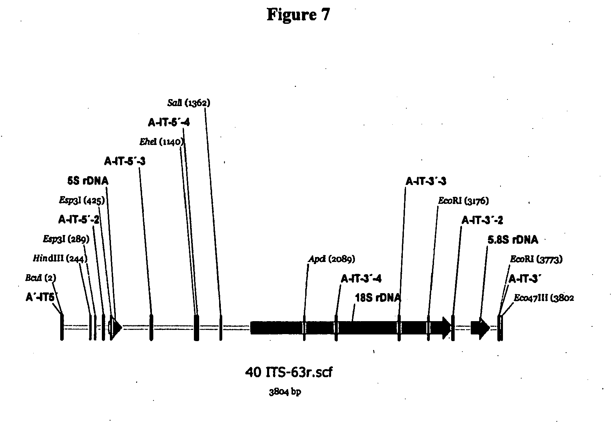

- **Figure 7:**: Schematic drawing of the ITS-5S-ETS-18S-IST-5,8SITS region of the rDNA. Restriction sites for endonucleases are indicated.

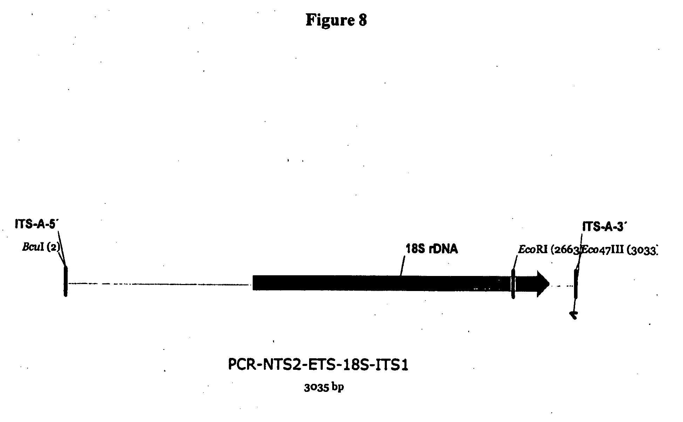

- **Figure 8:**: Schematic drawing of NTS2-ETS-18s-ITS1. Restriction sites for endonucleases are indicated.

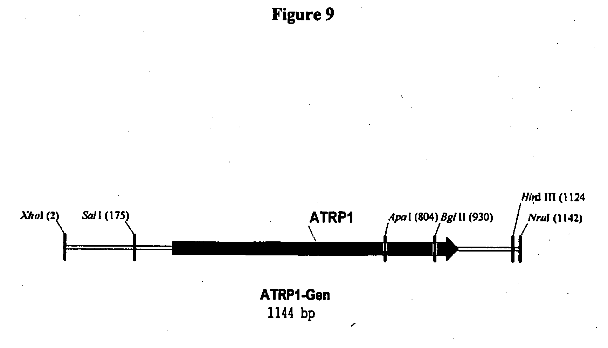

- **Figure 9:**: Schematic drawing of the selection marker gene *ATRP1*. Restriction sites for endonucleases are indicated.

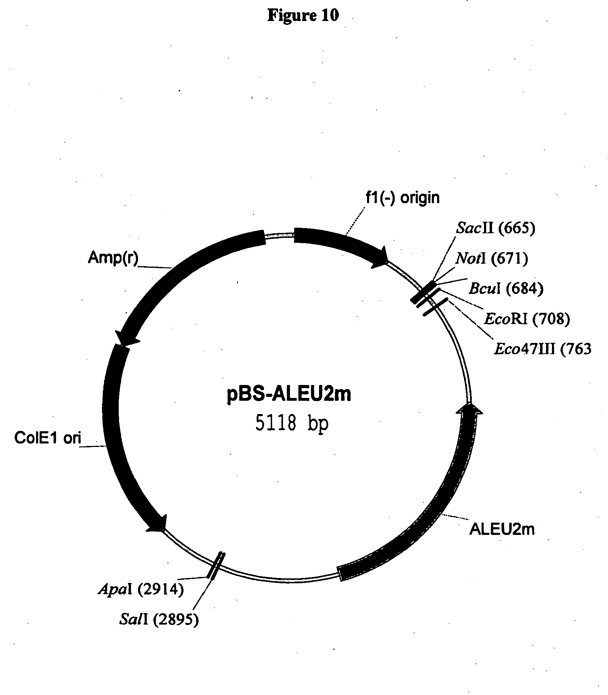

- **Figure 10:**: Schematic drawing of the selection marker gene *ALEU2* in a pBS-plasmid. Restriction sites for endonucleases are indicated.

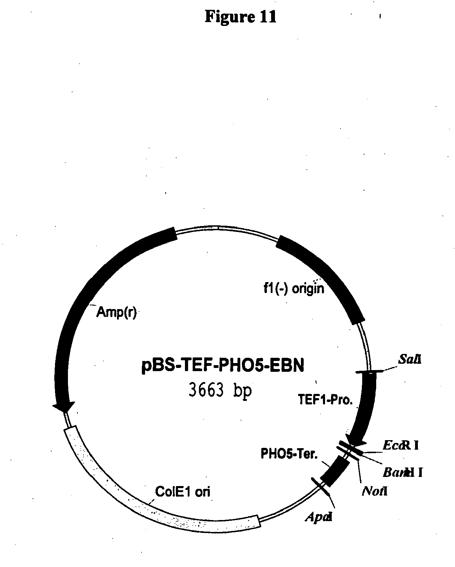

- **Figure 11:**: Schematic drawing of the *TEF*1-promoter expression cassette in pBS-plasmid (*Sal*I*-Apa*I). Restriction sites for endonucleases are indicated.

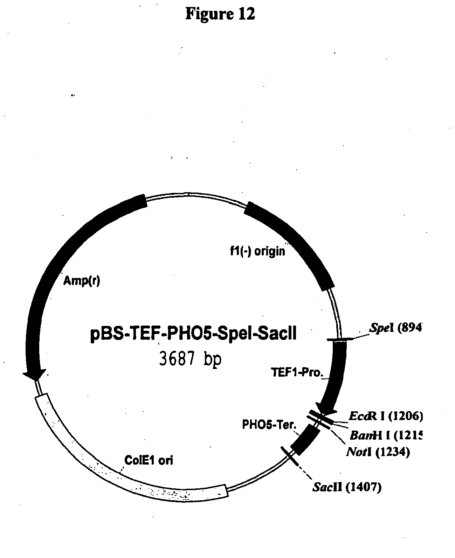

- **Figure 12:**: Schematic drawing of the -*TEF*1-promoter expression cassette in pBS-plasmid (*Spe*I (*Bcu*I)*-Sal*I). Restriction sites for endonucleases are indicated.

## Claims

1. A polynucleotide comprising functionally linked:
(a) an autonomously replicating nucleic acid sequence element comprising a CEN element (GI number: 406851), a 2µm (micron) element (GI number: 3296) and a nucleic acid sequence being at least 70 % identical thereto;
(b) a nucleic acid sequence comprising a ribosomal DNA (rDNA) gene;
(c) a nucleic acid sequence encoding a selection marker; and
(d) a nucleic acid sequence comprising an expressible nucleic acid sequence encoding a polypeptide and a promoter nucleic acid sequence governing the expression of said expressible sequence.

2. The polynucleotide of claim 1, wherein said rDNA gene is selected from any one of NTS2-ETS-18S-ITS1 (SEQ ID NO: 1), 25S rDNA (SEQ ID NO: 2), 18S rDNA (SEQ ID NO: 3), NTS-18S (SEQ ID NO: 4), NTS2-ETS-18S-ITS1 (SEQ ID NO: 5) or has a nucleic acid sequence being at least 70 % identical thereto.

3. The polynucleotide of claim 1 or claim 2, wherein said selection marker is selected from the group consisting *of ALEU2* gene (GI number: 21436710), *AILV1* gene (GI number: 2687748), *URA3* gene (GI number: 406851), *hph* gene, *kan* gene or *ATRP1* gene (SEQ ID NO: 6).

4. The polynucleotide of any one of claims 1 to 3, wherein said promoter is selected from any one of *TEF1* (GI number: 620041), *FMD* (SQ ID NO: 8), MOX (SEQ ID NO: 8), *TPS*1 (SEQ ID NO: 9), *AHSB4m* (GI number: 27550960), GAA (GI number: 600384), *ATAL* (GI number:17382031), AACP50 (GI number: 17382027) or ALIP (GI number: 58651818 or has a nucleic acid sequence being at least 70 % identical thereto.

5. The polynucleotide of any one of claims 1 to 4, wherein said expressible nucleic acid sequence encodes a secreted protein.

6. The polynucleotide of claim 5, wherein said secreted protein is a enzyme, growth factor, cytokine, survival factor, clotting factor, cytokine or growth factor receptor, structural protein of the extracellular matrix.

7. The polynucleotide of any one of claims 1 to 6, wherein the nucleic acid sequences (a) to (d) are arranged in 5' to 3' orientation starting with (a) at the 5'end and ending with (d) at the 3'end of the said polynucleotide.

8. A vector comprising the polynucleotide of any one of claims 1 to 7.

9. The vector of claim 8, wherein said vector has a nucleic acid sequence as shown in SEQ ID NO: 10.

10. A host cell comprising the polynucleotide of any one of claims 1 to 7 or the vector of claim 8 or 9.

11. The host cell of claim 10, wherein the cell is a yeast cell.

12. The host cell of claim 11, wherein said yeast is *Hansenula polymorpha* or *Arxula adeninivorans.*

13. A method for the manufacture of a polypeptide comprising the steps of:
(a) expressing the polynucleotide of any one of claims 1 to 7 or the vector of claim 8 or 9 in the host cell of any one of claims 10 to 12; and
(b) recovering from the said host cell the polypeptide encoded by the expressible nucleic acid sequence.

14. A method for the manufacture of a pharmaceutical composition comprising the steps of the method of claim 13 and the further step of formulating the polypeptide in a pharmaceutically acceptable manner.

15. Kit comprising the polynucleotide of any one of claims 1 to 7, the vector of claim 8 or 9 and/or the host cell of any one of claims 10 to 12.

16. Collection of vectors comprising a nucleic acid sequence comprising parts (a)-(d) of claim 1
wherein said nucleic acid sequences can be assembled resulting in a polynucleotide comprising at least one, at least two or at least three of the nucleic acid sequences referred to in any one of (a) to (c) and the nucleic acid sequence referred to in (d).

17. The Collection of claim 16, wherein the nucleic acid sequences can be assembled by use of any one of the restriction enzymes *BcuI, Eco47III, Sal1, EcoRI,* or *SacII.*

18. Kit comprising the collection of claim 16 or 17 and suitable means which allow assembling of the said nucleic acid sequences.

## Patentansprüche

1. Polynucleotid, das funktionell miteinander verknüpft Folgendes umfasst:
(a) ein autonom replizierendes Nucleinsäure-Sequenzelement, das ein CEN-Element (GI-Nummer: 406851), ein 2-µm-Element (GI-Nummer: 3296) und eine zu wenigstens 70% damit identische Nucleinsäuresequenz umfasst;
(b) eine Nucleinsäuresequenz, die ein Gen der ribosomalen DNA (rDNA) umfasst;
(c) eine Nucleinsäuresequenz, die einen Selektionsmarker codiert; und
(d) eine Nucleinsäuresequenz, die eine exprimierbare Nucleinsäuresequenz, welche ein Polypeptid codiert, und eine Promotornucleinsäuresequenz, die die Expression der exprimierbaren Sequenz steuert, umfasst.

2. Polynucleotid gemäß Anspruch 1, wobei das rDNA-Gen aus einem der Gene NTS2-ETS-18S-ITS1 (SEQ ID Nr. 1), 25S-rDNA (SEQ ID Nr. 2), 18S-rDNA (SEQ ID Nr. 3), NTS-18S (SEQ ID Nr. 4), NTS2-ETS-18S-ITS1 (SEQ ID Nr. 5) ausgewählt ist oder eine zu wenigstens 70% damit identische Nucleinsäuresequenz aufweist.

3. Polynucleotid gemäß Anspruch 1 oder 2, wobei der Selektionsmarker aus der Gruppe ausgewählt ist, die aus dem *ALEU2-Gen* (GI-Nummer: 21436710), *AILVI-Gen* (GI-Nummer: 2687748), *URA3-Gen* (GI-Nummer: 406851), *hph-Gen, kan-Gen* oder ATRP1-Gen (SEQ ID Nr. 6) besteht.

4. Polynucleotid gemäß einem der Ansprüche 1 bis 3, wobei der Promotor aus einem der Promotoren *TEF1* (GI-Nummer: 620041), *FMD* (SEQ ID Nr. 8), MOX (SEQ ID Nr. 8), *TPS1* (SEQ ID Nr. 9), *AHSB4m* (GI-Nummer: 27550960), GAA (GI-Nummer: 600384), *ATAL* (GI-Nummer: 17382031), AACP50 (GI-Nummer: 17382027) oder ALIP (GI-Nummer: 58651818 ausgewählt ist oder eine zu wenigstens 70% damit identische Nucleinsäuresequenz aufweist.

5. Polynucleotid gemäß einem der Ansprüche 1 bis 4, wobei die exprimierbare Nucleinsäuresequenz ein zu sezernierendes Protein codiert.

6. Polynucleotid gemäß Anspruch 5, wobei das zu sezernierende Protein ein Enzym, ein Wachstumsfaktor, ein Cytokin, ein Überlebensfaktor, ein Gerinnungsfaktor, ein Cytokin- oder Wachstumsfaktorrezeptor oder ein Strukturprotein für die extrazelluläre Matrix ist.

7. Polynucleotid gemäß einem der Ansprüche 1 bis 6, wobei die Nucleinsäuresequenzen (a) bis (d) in 5'→3'-Orientierung angeordnet sind, beginnend mit (a) am 5'-Ende und endend mit (d) am 3'-Ende des Polynucleotids.

8. Vektor, der das Polynucleotid gemäß einem der Ansprüche 1 bis 7 umfasst.

9. Vektor gemäß Anspruch 8, wobei der Vektor eine Nucleinsäuresequenz aufweist, wie sie in SEQ ID Nr. 10 gezeigt ist.

10. Wirtszelle, die das Polynucleotid gemäß einem der Ansprüche 1 bis 7 oder den Vektor gemäß Anspruch 8 oder 9 umfasst.

11. Wirtszelle gemäß Anspruch 10, wobei die Zelle eine Hefezelle ist.

12. Wirtszelle gemäß Anspruch 11, wobei es sich bei der Hefe um *Hansenula polymorpha* oder *Arxula adeninivorans* handelt.

13. Verfahren zur Herstellung eines Polypeptids, das die folgenden Schritte umfasst:
(a) Exprimieren des Polynucleotids gemäß einem der Ansprüche 1 bis 7 oder des Vektors gemäß Anspruch 8 oder 9 in der Wirtszelle gemäß einem der Ansprüche 10 bis 12; und
(b) Gewinnen des Polypeptids, das von der exprimierbaren Nucleinsäuresequenz codiert wird, aus der Wirtszelle.

14. Verfahren zur Herstellung einer pharmazeutischen Zusammensetzung, das die Schritte des Verfahrens gemäß Anspruch 13 und den weiteren Schritt des Zubereitens des Polypeptids in einer pharmazeutisch annehmbaren Weise umfasst.

15. Kit, der das Polynucleotid gemäß einem der Ansprüche 1 bis 7, den Vektor gemäß Anspruch 8 oder 9 und/oder die Wirtszelle gemäß einem der Ansprüche 10 bis 12 umfasst.

16. Kollektion von Vektoren, die eine Nucleinsäuresequenz umfassen, die die Teile (a) bis (d) von Anspruch 1 umfasst, wobei die Nucleinsäuresequenzen zu einem Polynucleotid zusammengesetzt werden können, das wenigstens eine, wenigstens zwei oder wenigstens drei der Nucleinsäuresequenzen, die in (a) bis (c) genannt sind, und die Nucleinsäuresequenz, die in (d) genannt ist, umfasst.

17. Kollektion gemäß Anspruch 16, wobei die Nucleinsäuresequenzen unter Verwendung eines der Restriktionsenzyme *BcuI, Eco*47III, *Sal*I, *Eco*RI oder SacII zusammengesetzt werden können.

18. Kit, der die Kollektion gemäß Anspruch 16 oder 17 und geeignete Mittel, die das Zusammensetzen der Nucleinsäuresequenzen ermöglichen, umfasst.

## Revendications

1. Polynucléotide comprenant de manière fonctionnellement liée :
(a) un élément de séquence d'acide nucléique à réplication autonome comprenant un élément CEN (numéro GI : 406851), un élément de 2 µm (micron) (numéro GI : 3296) et une séquence d'acide nucléique qui lui est identique à au moins 70 % ;
(b) une séquence d'acide nucléique comprenant un gène d'ADN ribosomique (ADNr) ;
(c) une séquence d'acide nucléique codant pour un marqueur de sélection ; et
(d) une séquence d'acide nucléique comprenant une séquence d'acide nucléique exprimable codant pour un polypeptide et une séquence d'acide nucléique de promoteur gouvernant l'expression de ladite séquence exprimable.

2. Polynucléotide selon la revendication 1, dans lequel ledit gène d'ADNr est choisi parmi l'un quelconque des suivants NTS2-ETS-18S-ITS1 (SEQ ID NO : 1), 25S ADNr (SEQ ID NO : 2), 18S ADNr (SEQ ID NO : 3), NTS-18S (SEQ ID NO : 4), NTS2-ETS-18S-ITS1 (SEQ ID NO : 5) ou a une séquence d'acide nucléique qui leur est au moins identique à 70 %.

3. Polynucléotide selon la revendication 1 ou la revendication 2, dans lequel ledit marqueur de sélection est choisi dans le groupe constitué par le gène *ALEU2* (numéro GI : 21436710), le gène *AILV1* (numéro GI : 2687748), le gène *URA3* (numéro GI : 406851), le gène hph, le gène kan ou le gène *ATRP1* (SEQ ID NO : 6).

4. Polynucléotide selon l'une quelconque des revendications 1 à 3, dans lequel ledit promoteur est choisi parmi l'un quelconque des suivants TEF1 (numéro GI : 620041), *FMD* (SEQ ID NO : 8), MOX (SEQ ID NO : 8), *TPS1* (SEQ ID NO : 9), AHSB4m (numéro GI : 27550960), GAA (numéro GI : 600384), ATAL (numéro GI : 17382031), AACP50 (numéro GI : 17382027) ou ALIP (numéro GI : 58651818) ou a une séquence d'acide nucléique qui lui est au moins identique à 70 %.

5. Polynucléotide selon l'une quelconque des revendications 1 à 4, dans lequel ladite séquence d'acide nucléique exprimable code pour une protéine sécrétée.

6. Polynucléotide selon la revendication 5, dans lequel ladite protéine sécrétée est une enzyme, un facteur de croissance, une cytokine, un facteur de survie, un facteur de coagulation, une cytokine ou un récepteur de facteur de croissance, une protéine structurelle de la matrice extracellulaire.

7. Polynucléotide selon l'une quelconque des revendications 1 à 6, dans lequel les séquences d'acide nucléique (a) à (d) sont agencées dans la direction 5' à 3' commençant avec (a) à l'extrémité 5' et se terminant avec (d) à l'extrémité 3' dudit polynucléotide.

8. Vecteur comprenant le polynucléotide de l'une quelconque des revendications 1 à 7.

9. Vecteur selon la revendication 8, dans lequel ledit vecteur a une séquence d'acide nucléique comme montré dans SEQ ID NO : 10.

10. Cellule hôte comprenant le polynucléotide de l'une quelconque des revendications 1 à 7 ou le vecteur de la revendication 8 ou 9.

11. Cellule hôte selon la revendication 10, dans laquelle la cellule est une cellule de levure.

12. Cellule hôte selon la revendication 11, dans laquelle ladite levure est *Hansenula polymorpha* ou *Arxula adeninivorans.*

13. Procédé de fabrication d'un polypeptide comprenant les étapes consistant à :
(a) exprimer le polynucléotide de l'une quelconque des revendications 1 à 7 ou le vecteur de la revendication 8 ou 9 dans la cellule hôte de l'une quelconque des revendications 10 à 12 ; et
(b) récupérer de ladite cellule hôte le polypeptide codé par la séquence d'acide nucléique exprimable.

14. Procédé de fabrication d'une composition pharmaceutique comprenant les étapes du procédé de la revendication 13 et l'étape supplémentaire consistant à formuler le polypeptide de manière pharmaceutiquement acceptable.

15. Nécessaire comprenant le polynucléotide de l'une quelconque des revendications 1 à 7, le vecteur de la revendication 8 ou 9 et/ou la cellule hôte de l'une quelconque des revendications 10 à 12.

16. Collection de vecteurs comprenant une séquence d'acide nucléique comprenant
les éléments (a) à (d) de la revendication 1
dans laquelle lesdites séquences d'acide nucléique peuvent être assemblées ce qui aboutit à un polynucléotide comprenant au moins une, au moins deux ou au moins trois des séquences d'acide nucléique désignées dans l'un quelconque de (a) à (c) et de la séquence d'acide nucléique indiquée dans (d).

17. Collection selon la revendication 16, dans laquelle les séquences d'acide nucléique peuvent être assemblées à l'aide de l'une quelconque des enzymes de restriction *BcuI, Eco*47III, *SalI, Eco*RI ou *Sac*II.

18. Nécessaire comprenant la collection de la revendication 16 ou 17 et un moyen approprié qui permet l'assemblage desdites séquences d'acide nucléique.
